# EUROPÄISCHE PATENTSCHRIFT

(11) **EP 0 322 453 B1**
(45) Veröffentlichungstag und Bekanntmachung des Hinweises auf die Patenterteilung: **01.09.1993**
(21) Anmeldenummer: 88907192.4
(22) Anmeldetag: 11.07.1988
(51) Int. Cl.: A61B 5/00, A61M 5/14

(54) **VORRICHTUNG ZUR ENTNAHME VON KÖRPERFLÜSSIGKEITEN**
DEVICE FOR WITHDRAWAL OF BODY FLUIDS
DISPOSITIF DE PRELEVEMENT DE FLUIDES CORPORELS

(30) Priorität: 09.07.1987 AT 1732/87
(43) Veröffentlichungstag der Anmeldung: 05.07.1989
(73) Patentinhaber: AVL Medical Instruments AG, 8207 Schaffhausen (CH)
(72) Erfinder: SKRABAL, Falko, A-8010 Graz (AT)
(74) Vertreter: Krause, Walter, Dr. Dipl.-Ing.
(86) Internationale Anmeldenummer: AT8800051
(87) Internationale Veröffentlichungsnummer: WO8900397

(56) Entgegenhaltungen:
- EP-A- 0 110 687
- EP-A- 0 154 002
- EP-A- 0 273 258
- WO-A-84/04033
- DE-A- 3 040 168
- DE-A- 3 307 810
- DE-B- 2 209 322
- US-A- 4 127 111
- US-A- 4 573 968

## Beschreibung

Die Erfindung betrifft eine Vorrichtung zur Entnahme von Körperflüssigkeiten, mit einer Entnahmeeinrichtung, einer Saugeinrichtung, einer Druckeinrichtung sowie einem Speichersystem zur Aufnahme der Körperflüssigkeit, welches voneinander trennbare Teilbereiche aufweist, welche die in bestimmten Zeitabschnitten gesammelten Flüssigkeitsfraktionen aufnehmen und eine eindeutige zeitliche Zuordnung der einzelnen Flüssigkeitsfraktionen zulassen, wobei die Entnahmeeinrichtung als zweilumige Nadel ausgeführt ist, deren ein Lumen mit der zuführenden und deren anderes Lumen mit der abführenden Leitung verbunden ist, wobei die Saugeinrichtung mit der abführenden Leitung für die zu gewinnende Körperflüssigkeit und die zuführende Leitung mit der Druckeinrichtung in, Verbindung steht.

Zur Entnahme von Körperflüssigkeiten, wie Blut, Gewebeflüssigkeit, Flüssigkeit aus Körperhöhlen oder auch Harn, sind in dar Medizin unterschiedliche Vorrichtungen bekannt, wobei vor allem Kolbenspritzen, Pipetten sowie am Entnahmeort verbleibende Kanülen und Katheter Verwendung finden.

Bei vielen Untersuchungen muß der bzw. die jeweils für die Untersuchung wichtigen, Parameter der Körperflüssigkeit über einen gewissen Zeitraum gemessen oder kontrolliert werden, wobei im Hinblick auf die sich oft rasch ändernden Parameter zumeist in kurzen Abständen Körperflüssigkeit entnommen und analysiert werden muß.

So ist es beispielsweise bei gewissen Hormonuntersuchungen notwendig, stündlich Blutproben zu nehmen, was insbesonders in den Nachtstunden für Patient und Arzt äußerst unangenehm ist. Als Beispiel für die gewünschte Blutabnahme im Sinne eines Tagesprofils seien zahlreiche hormonelle Störungen angeführt : Morbus Cushing mit einer gestörten Gortisontagesrhytmik, Störungen des Wachstums und der Geschlechtsreife mit Ausbleiben der nächtlichen Hormongipfel von Wachstumshormonen bzw. Geschlechtshormonen und vieles andere mehr.

Ein weiteres wichtiges Anwendungsbeispiel ist die Pharmaindustrie, wo es wichtig ist, die Blut- und Gewebespiegel von Pharmaka bzw. deren Wirkung über 24 Stunden zu registrieren`

Natürlich ist es in einigen Fällen auch möglich, über liegende arterielle oder venöse Zugänge Blut zu gewinnen, wobei zumindest die mehrmals täglich wiederkehrende unangenehme Stechbelastung entfällt; die störende Manipulation am Patienten verbleibt jedoch.

In diesem Zusammenhang ist eine aus der nachveröffentlichten EP 0 273 258 A1 [Art 54(3)] bekanntgewordene Anordnung zur Untersuchung eines flüssigen Mediums von Interesse, welche eingangs zitiert wurde. Die daraus bekannte Vorrichtung weist einen zweilumigen Katheter auf, welcher in den Blutstrom eines Patienten eingeführt werden kann. Das äußere Lumen, dessen Mündung in den Blustrom reicht, ist über einen Infusionsschlauch sowie eine Vorrichtung zur Umkehr der Strömungsrichtung, insbesondere eine umsteuerbare Pumpe, an einem Behälter mit einer Infusionslösung angeschlossen. Das innere Lumen, dessen Mündung sich innerhalb des äußeren Lumens befindet , ist über einen Infusionsschlauch mit einem Meßkanal verbunden, in welchem sich ein Meß- und ein Referenzsensor befinden.

Weiters ist aus der WO-A 84/04033 ein tragbares Entnahmegerät für Blut und ander Flüssigkeiten sowie ein Verfahren zur Blut- und Flüssigkeitsentnahme bekanntgeworden. Das Entnahmegerät weist eine einlumige Entnahmenadel auf, über welche die entnommene Körperflüssigkeit einem Speicher zugeführt werden kann, in welchem mehrere Körperflüssigkeitsproben speicherbar sind. Weiters ist eine Spül- und Füllvorrichtung für eine Pumpe, den einlumigen Katheter und das von der Pumpe zum Speicher führende Kanalsystem vorgesehen.

Aufgabe der Erfindung ist es, ausgehend von einer eingangs genannten Vorrichtung, eine Vorrichtung zur Entnahme von beliebigen Körperflüssigkeiten vorzuschlagen, welche technisch einfach und Kostengünstig ausgeführt ist, wobei als weitere Aufgabe der Erfindung eine einfache Reinigung der Vorrichtung gewährleistet sein soll.

Erfindungsgemäß wird diese Aufgabe dadurch gelöst, daß die Saugeinrichtung und die Druckeinrichtung durch eine Kolbenpumpe realisiert sind, welche druckseitig ein mit der zuführenden Leitung verbundenes Reservoir für die in den Körper einzubringende Flüssigkeit und saugseitig einen mit dem Speichersystem verbundenen Saugraum aufweist. Bei allen Untersuchungen, bei welchen aus verschiedensten Gründen eine On-Line-Analyse der gewonnenen Körperflüssigkeit nicht möglich ist, kann mit Hilfe der erfindungsgemäßen Vorrichtung auch nach über einen längeren Zeitraum erfolgter Probenentnahme, eine dein Entnahmezeitpunkt zuordenbare Analyse der einzelnen interessierenden Parameter durchgeführt werden.

Als Entnahmeeinrichtung wird dabei eine im Blutstrom liegende zweilumige Nadel verwendet. Für Anwendungen der Vorrichtung direkt im Gewebe kann durch ein Lumen der zweilumigen Nadel eine Perfusionsflüssigkeit direkt in das Gewebe eingebracht und nach deren teilweisen Equilibration mit den interessierenden Parametern des Gewebes fraktionsweise gesammelt und anschließend auf die interessierenden Parameter untersucht werden. Zweckmäßig dabei ist es, endogene oder exogene Marker zu verwenden, um den Grad der Wechselwirkung der Perfusionsflüssigkeit mit dem Gewebe festzustellen und bei der nachfolgenden Analyse zu berücksichtigen. Vorteilhafterweise ist hier nur eine teilweise Equilibration notwendig, da die Perfusionsflüssigkeit sowohl auf den interessierenden Parameter als auch auf die endogenen oder exogenen Markereigenschaften analysiert wird. Bei der Anwendung der Vorrichtung im Blut kann über ein Lumen der zweilumigen Nadel ein Mittel gegen die Blutgerinnung, z.B. Heparin apliziert werden, wobei über das andere Lumen die Blutentnahme erfolgt.

Für verschiedene Anwendungen der Vorrichtung, bei welchen die Saug- und Druckvolumina nicht gleich groß sind, können mit einem Faltenbalg versehen Ausgleichsgefäße vorgesehen sein, welche entweder mit dem druckseitigen oder saugseitigen Teil der Kolbenpumpe in Verbindung stehen.

Die Vorrichtung kann beispielsweise erfindungsgemäß einen Auffangbehälter mit vorzugsweise variablem Fassungsvermögen aufweisen, welcher über ein die Durchflußmenge steuerndes Organ mit dem Reservoir für die in den Körper einzubringende Flüssigkeit verbunden ist. Damit können bei voller Saugleistung auch kleinere Flüssigkeitsmengen zugeführt werden. So liegt beispielsweise bei Anwendungen, wo eine Zufuhr von Heparin angebracht ist, das Verhältnis von entnommenen Blut zu zugeführtem Heparin zwischen 10 : 1 und 500 : 1.

In einer ersten Ausführungsvariante kann dabei als Speichersystem ein die Entnahmeeinrichtung mit der Saugeinrichtung verbindender Schlauch vorgesehen sein, wobei zur Trennung der einzelnen Flüssigkeitsfraktionen im Bereich des Schlaucheinganges eine Stetereinrichtung angeordnet ist, über welche in definierten Zeitabständen ein die einzelnen Flüssigkeitsfraktionen separierendes gasförmiges Medium in den Schlauch einbringbar ist. Als Steuereinrichtung kann beispielsweise ein Ventil fungieren, welches das Speichersystem nach definierten Zeitabschnitten mit der Außenluft in Verbindung bringt, wodurch eine Luftblase in den Schlauch des Speicherystems eingesaugt werden kann und so die einzelnen Flüssigkeitsfraktionen separiert werden.

Eine andere Ausführungsvariante sieht vor, daß die Entnahmeeinrichtung in Anlehnung an die aus der WO-A 8404033 bekannte Vorrichtung mit einem Verteiler in Verbindung steht, wobei für die einzelnen trennbaren Teilbereiche eines Speichersystems vom Verteiler wegführende, parallel geschaltete Zweigleitungen vorgesehen sind und eine Steuereinrichtung jeweils eine der Zweigleitungen mit der Entnahmeeinrichtung verbindet. Vorteilhafterweise kommt bei dieser Ausführung jede Flüssigkeitsfraktion nur mit einer bestimmten Zweigleitung in Konakt, sodaß ein Vermischen oder Verschleppen von Bestandteilen anderer Flüssigkeitsfraktionen weitgehend ausgeschlossen wird.

Die zweilumige Nadel kann beispielsweise eine innere, mit der abführenden Leitung verbundene Kanüle aufweisen, welche von einer zum Gewebe Öffnungen aufweisenden äußeren Kanüle umgeben ist, welche mit der zuführenden Leitung verbunden ist. Die genannte Vorrichtung eignet sich z.B. dazu, aus den gewonnenen Flüssigkeitsfraktionen den zum jeweiligen Entnahmezeitpunkt der einzelnen Fraktionen vorherrschenden Gewebespiegel eines Hormons, eines Substrates, z.B. des Blutzuckers oder eines Pharmakons zu bestimmen.

Im Hinblick auf eine vereinfachte Reinigung und Handhabung der erfindungsgemäßen Vorrichtung ist vorgesehen, daß der Antrieb der Saug- und Druckeinrichtung, sowie jener der Steuereinrichtung des Speichersystems gemeinsam mit der zugehörigen Steuerelektronik in einem Grundgehäuse angeordnet ist und das Speichersystem zur Aufnahme der Körperflüssigkeit, die Saug- und Druckeinrichtung sowie deren Verbindungsleitungen in einer vom Grundgehäuse abnehmbaren Kassette angeordnet sind. Alle mit der Körperflüssigkeit in Berührung kommenden Teile der Vorrichtung können mit der Kassette entfernt und durch eine neue ersetzt werden.

Als besonders vorteilhaft hat es sich dabei erwiesen, wenn die Kassette U-förmig ausgebildet ist, wobei an beiden Seitenflächen des Grundgehäuses je ein Teil der Kassette mit jeweils einer Vielzahl einzelner Zweigleitungen vorgesehen ist, wobei alle Zweigleitungen über eine Sammelleitung mit der Saugeinrichtung verbunden sind. Beispielsweise können Zweigleitungen für insgesamt 48 Flüssigkeitsfraktionen vorhanden sein, sodaß bei einer halbstündlichen Flüssigkeitsentnahme ein Meßbetrieb über 24 Stunden aufrecht erhalten werden kann.

Da einzelne gewinnbare Körperflüssigkeiten bzw. deren Inhaltsstoffe sehr temperaturempfindlich sind, ist vorgesehen, daß die das Speichersystem aufnehmende Kassette bzw. deren Teile mit einer regelbaren Kühleinrichtung in Verbindung stehen, welche über ein Gehäuse zur Aufnahme eines Kühlgels verfügt und in der dem Speichersystem zugewandten Wand vorzugsweise gleichmäßig verteilte Öffnungen aufweist., über deren durch einen Thermostat steuerbaren Öffnungsquerschnitte Kühlluft zum Speichersystem zutritt. Damit können die einzelnen Flüssigkeitsfraktionen bis zu ihrer Analyse auf die für sie notwendige Temperatur gekühlt werden. Über den steuerbaren Öffnungsquerschnitt der Öffnungen kann die sich verringernde Kühlleistung des Kühlgels ausgeglichen werden.

Zur weiteren Vereinfachung der erfindungsgemäßen Vorrichtung kann auch nur ein Antriebsmotor vorgesehen sein, welcher über ein Spindelgetriebe die Saug- und Druckeinrichtung betätigt und über ein Untersetzungsgetriebe und ein Klinkenrad eine Nockenwelle antreibt, deren Nocken mit am Grundgehäuse befestigten Quetschhebeln der Steuereinrichtung zusammenwirken, wobei jeweils ein durch einen Nocken angehobener Quetschhebel eine der Zweigleitungen freigibt. Auf zusätzliche aufwendige Mechanik kann dabei verzichtet werden. Es ist natürlich auch möglich, mit der Nockenwelle zusammenwirkende Abklemmvorrichtungen in der abnehmbaren Kassette vorzusehen.

Zur Auswertung der einzelnen Fraktionen müssen diese aus der Vorrichtung entnommen und in einzelne Eprouvetten abgefüllt werden. Zu diesem Zweck ist erfindungsgemäß vorgesehen, daß der Antrieb der Saug- und Druckeinrichtung zur Entnahme der einzelnen Flüssigkeitsfraktionen aus dem Speichersystem umschaltbar ist. Die Saugeinrichtung funktioniert dann als Druckeinrichtung und gibt die einzelnen Flüssigkeitsfraktionen über das Entnahmesystem - beispielsweise die Entnahmenadel oder Entnahmekanüle - frei.

Es kann natürlich zur Entnahme der einzelnen Flüssigkeitsfraktionen auch ein mit der Saug- und Druckeinrichtung und der Steuereinrichtung des Speichersystems zusammenwirkendes Handrad bzw. Kurbel vorgesehen sein.

Um ein Vermischen der einzelnen Flüssigkeitsfraktionen nachhaltig zu vermeiden, ist erfindungsgemäß vorgesehen, daß der Durchmesser der die Flüssigkeitsfraktionen aufnehmenden Zweigleitungen bzw. des Schlauches klein bemessen ist, sodaß zwischen den Innenwänden des Speichersystems und der Körperflüssigkeit wirkende Adhäsionskräfte größer sind, als vor, außen auf die Vorrichtung wirkende Kräfte, wie Schwerkraft oder zusätzliche Beschleunigungskräfte. Der Durchmesser, der die Flüssigkeitsfraktionen enthaltenden Leitungen sollte so gewählt werden, daß die einzelnen Fraktionen durch Adhäsionskräfte fixiert werden und unabhängig von der Lage der einzelnen Leitungen im Raum oder auf darauf wirkende Beschleunigungskräfte an der einmal vorgegebenen Stelle in Speichersystem verbleiben.

Schließlich kann die Steuerelektronik über ein Display zur Eingabe eines Zeitsteuerprogramms verfügen, womit die einzelnen Flüssigkeitsfraktionen zu individuellen vorgebbaren Zeitpunkten entnommen werden können.

Im folgenden wird die Erfindung anhand von Zeichnungen näher erläutert. Es zeigen:
- Fig. 1: eine Vorrichtung nach der Erfindung,
- Fig. 1a: Detail A aus Fig. 1,
- Fig. 2: eine andere Vorrichtung nach der Erfindung,
- Fig. 3: eine Ausführungsvariante nach Fig. 1,
- Fig. 4: einen Schnitt nach der Linie IV-IV in Fig. 3, sowie
- Fig. 5: eine Draufsicht in Richtung Pfeil V in Fig. 3 bei teilweise entfernter Kühleinrichtung.

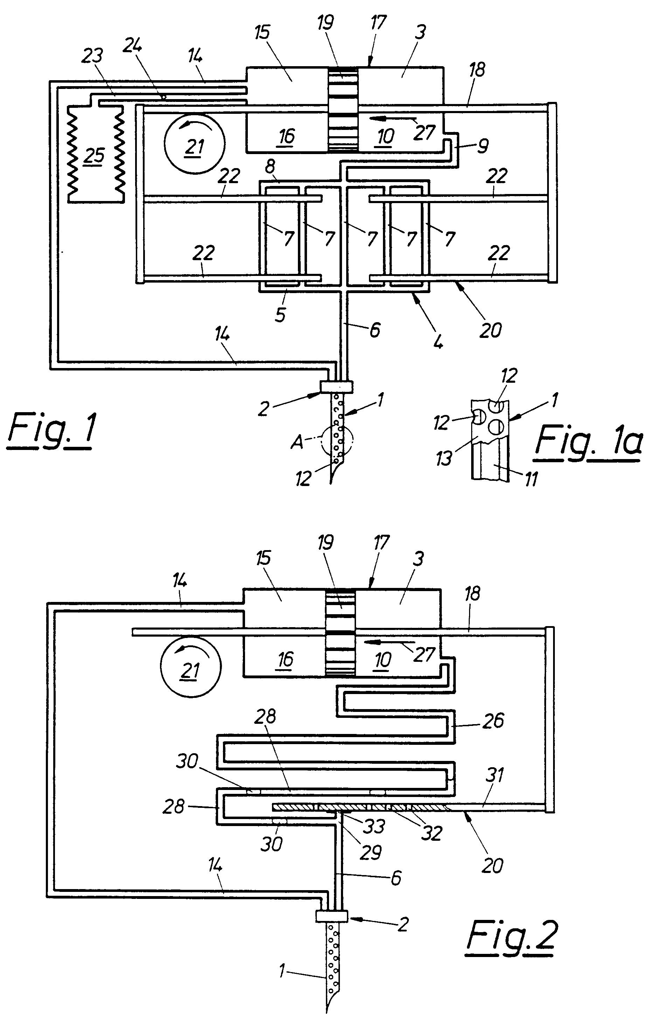

Eine Vorrichtung zur Entnahme von Körperflüssigkeiten ist schematisch in Fig. 1 dargestellt. Zwischen einer als zweilumige Nadel 1 ausgebildeten Entnahmeeinrichtung 2 und einer Saugeinrichtung 3 befindet sich ein Speichersystem 4 zur Aufnahme der gewonnenen Flüssigkeitsfraktionen. Ausgehend von einem Verteiler 5, welcher mit der abführenden Leitung 6 der Entnahmeeinrichtung 2 in Verbindung steht , umfaßt das Speichersystem 4 eine Reihe von parallelgeschalteten Zweigleitungen 7, welche über eine Sammelleitung 8 und einer davon ausgehenden Verbindungsleitung 9 mit dem Saugraum 10 der Saugeinrichtung 3 verbunden sind.

Wie aus dem in Fig. 1a dargestellten Detail A aus Fig. 1 ersichtlich, kann die Entnahmeeinrichtung 2 als zweilumige Suboutannade 1 ausgebildet sein, deren inneres Lumen 11 mit der abführenden Leitung 6 und deren äußeres, mit Öffnungen 12 versehenes Lumen 13 mit einer zuführenden Leitung 14 in Kontakt steht. Die zuführende Leitung 14 ist mit einem Reservoir 16 einer Druckeinrichtung 15 verbunden, aus welchem der Entnahmeeinrichtung 2 eine in den Körper einzubringende Flüssigkeit, beispielsweise ein Perfusat oder ein die Blutgerinnung hemmendes Mittel etc., zugeführt werden kann.

Bei einer Vorrichtung, welche lediglich zur Blutentnahme verwendet wird, braucht bei Verwendung einer zweilumigen Nadel die äußere Kanüle keine Öffnungen 12 aufweisen, da das Blut durch die vordere Öffnung der Nadel angesaugt wird. Die innere Kanüle 11, die nicht ganz an die Spitze der äußeren Kanüle 13 reicht, kann dazu verwendet werden, dem angesaugten Blut eine gerinnungshemmende Substanz, z.B. Heparin, beizumischen.

Einige wenige Öffnungen 12 im Bereich der Kanülenspitze könnten auch hier von Vorteil sein, um das Ansaugen von Blut auch darin zu gewährleisten, wenn z.B. die vordere Öffnung der Kanüle an der Gefäßwand anliegt.

Die Saugeinrichtung 3 und die Druckeinrichtung 15 werden durch eine Kolbenpumpe 17 realisiert, deren Saugraum 10 vom Reservoir 16 durch einen über einen Antriebsstempel 18 bewegbaren Kolben 19 getrennt ist.

Eine Steuereinrichtung 20 verbindet jeweils eine der Zweigleitungen 7 mit der abführenden Leitung 6, wobei die Steuereinrichtung 20 über eine - hier nur schematisch angedeutete - mechanische Kopplung mit dem Antriebsstempel 18 der Kolbenpumpe 17 in Verbindung steht und mit dieser über einen gemeinsamen Antrieb 21 betätigbar ist. Die Arme 22 der Steuereinrichtung 20 klemmen die nicht mit der Körperflüssigkeit zu beaufschlagenden Zweigleitungen 7 an beiden Enden ab.

Über eine weitere Leitung 23 in welcher sich ein die Durchflußmenge steuerndes Organ 24 befindet, ist ein Auffangbehälter 25 mit variablem Fassungsvermögen an das Reservoir 16 angeschlossen. Über das die Durchflußmenge steuernde Organ 24 kann bei voller Saugleistung die aus dem Reservoir 16 in die zuführende Leistung 14 Strömende Flüssigkeitsmenge variiert werden.

Bei Bewegung des Kolbens 19 - und damit der Steuereinrichtung 20 - in Richtung des Pfeiles 27 entsteht im Saugraum 10 ein Unterdruck, durch welchen Körperflüssigkeit in die von der Steuereinrichtung 20 freigegebene Zweigleitung gefördert wird. Bei Weiterbewegung des Kolbens und der Steuereinrichtung wird nach einiger Zeit die eine Zweigleitung von den Armen 22 der Steuereinrichtung 20 abgeklemmt und die in Pfeilrichtung nächste Zweigleitung 7 freigegeben. Zur Entleerung der einzelnen Flüssigkeitsfraktionen aus den Zweigleitungen 7 muß lediglich die Antriebseinrichtung umgeschaltet werden.

In allen folgenden Ausführungsvarianten sind gleiche Teile mit gleichen Bezugszeichen versehen. So zeigt Fig. 2 ein Ausführungsbeispiel. bei welchem das Speichersystem 4 als Schlauch 26 ausgebildet ist, welcher die Entnehmeeinrichtung 2 mit der Saugeinrichtung 3 verbindet. Zur Trennung der einzelnen Flüssigkeitsfraktionen 28 ist im Bereich des Schlaucheinganges 29 eine Steuereinrichtung 20 angeordnet, über welche in definierten Zeitabständen ein die Flüssigkeitsfraktionen separierendes Medium - in Form von Gasblasen 30 - eingebracht werden kann.

Das Steuerorgan 20 ist hier schematisch durch einem Arm 31 dargestellt, welcher Öffnungen 32 aufweist, die bei fortlaufender Bewegung des Steuerorganes in Richtung des Pfeiles 27 eine Öffnung 33 im Bereich des Schlaucheinganges 29 freigeben, wodurch kurzzeitig Umgebungsluft angesaugt wird.

Falls der Zutritt von Luft bzw. Luft-Sauerstoff unerwünscht ist, kann natürlich auch jedes andere, die einzelnen Flüssigkeitsfraktionen nicht beeinflussende Gas mit Hilfe eines derartigen Steuerorganes zugeführt werden, wobei lediglich ein Behälter und eine Zuleitung dafür vorgesehen sein müßte.

Sowohl in dem Ausführungsbeispiel nach Fig. 1 als auch in jenem nach Fig. 2 ist es natürlich auch möglich, der Steuereinrichtung 20 einen eigenen Antrieb zuzuordnen, beispielsweise könnte insbesondere in der Ausführung nach Fig. 2 ein Magnetventil Verwendung finden.

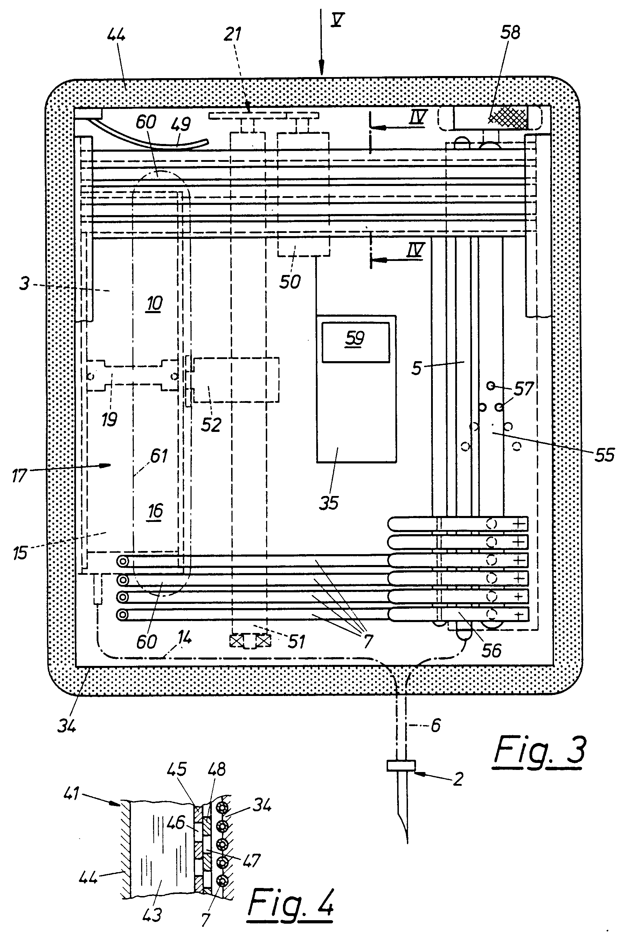

Die in den Fig. 3 bis 5 dargestellte Ausführungsvariante umfaßt ein Grundgehäuse 34, welches den Antrieb 21 der als Kolbenpumpe 17 ausgeführten Saug- und Druckeinrichtung und die zugehörige Steuerelektronik 35 aufnimmt, sowie eine vom Grundgehäuse 34 abnehmbare Kassette 36, in welcher das Speichersystem 4, die Saug- und Druckeinrichtung 3 und 15, sowie deren Verbindungsleitungen 5, 6, 8 und 14 angeordnet sind.

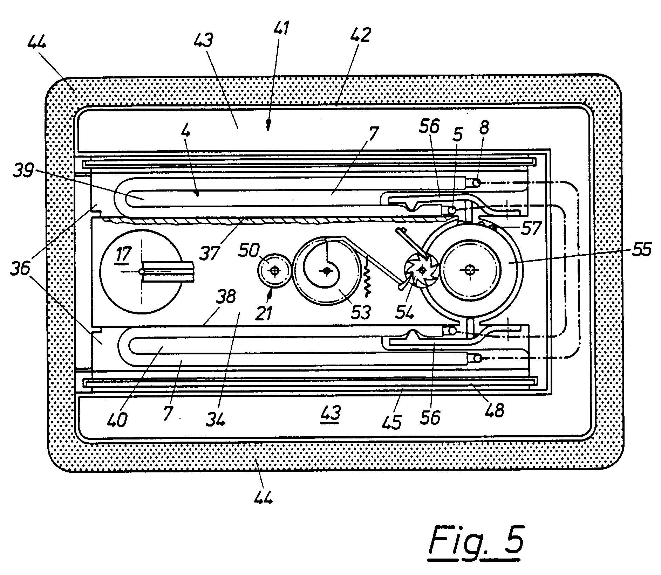

Wie aus Fig. 5 ersichtlich, ist die Kassette 36 U-förmig ausgebildet, wobei an beiden Seitenflächen 37, 38 des Grundgehäuses 34 je ein Teil 39, 40 der Kassette mit jeweils vierundzwanzig einzelnen Zweigleitungen 7 mit einem Fassungsvermögen von jeweils ca. 1 ml angeordnet ist. Es ist auch vorgesehen, Kassetten 36 mit Zweigleitungen 7 mit einem Fassungsvermögen von z.B. jeweils ½ ml oder 2 ml auszubilden sodaß eigene Kassetten für die Behandlung von Kindern und Erwachsenen zur Verfügung stehen. Die Vergrößerung des Volumens kann auf einfache Weise durch Erhöhung der Windungszahl der Zweigleitungen 7 erreicht werden, wobei der Leitungsquerschnitt klein gehalten werden kann.

Es ist natürlich auch möglich, für die einzelnen Flüssigkeitsfraktionen direkt in der Kassette 36 mitgeformte Kanäle vorzusehen, welche an einer Stelle eine Membran aufweisen, auf welche eine Abklemmeinrichtung wirksam wird.

Die das Speichersystem 4 aufnehmenden Teilkassetten 39, 40 werden von einer in den Fig. 4 und 5 dargestellten Kühleinrichtung 41 umgeben, in deren Gehäuse 42 sich ein Kühlgel 43 befindet. Um eine kontinuierliche Kühlung der Flüssigkeitsfraktionen in den Zweigleitungen 7 auf ca. 4° C zu gewährleisten, muß das Kühlgel auf minus 30° C abgekühlt werden und das Gehäuse 42 außen mit einer Isolierschicht 44 versehen sein. In der dem Speichersystem 4 zugewandten Wand 45 der Kühleinrichtung 41 sind gleichmäßig verteilte schlitzförmige Öffnungen 46 angeordnet, deren Öffnungsquerschnitt durch Verschieben einer mit korrespondierenden Öffnungen 47 ausgestatteten Blende 48 variiert werden kann. Die Wand 45 und die Blende 48 müssen aus einem Material geringer Wärmeleitfähigkeit bestehen und eine ausreichende Stärke aufweisen, um ein Unterschreiten der für die Flüssigkeitsfraktionen zulässigen Temperatur zu vermeiden.

Die Ansteuerung der Blende 48 kann über einen Thermostat 49, beispielsweise ein Bimetallelement, erfolgen, sodaß abhängig von der sinkenden Kühlleistung des Kühlgels für ausreichende Kühlung der Flüssigkeitsfraktionen gesorgt ist. Am Anfang bei sehr niedrigen Temperaturen des Kühlmediums, ist die Blende fast ganz geschlossen, um nur einen geringen Teil der Kälteleistung an die Flüssigkeitsfraktionen zu lassen, mit zunehmenden Kälteverlust im Kühlmedium wird dann die Blende entsprechend weiter geöffnet.

Die in den Fig. 3 bis 5 dargestellte Vorrichtung weist nur einen Antriebsmotor 50 auf, welcher über einen Mitnehmer 52 eines Spindelgetriebes 51 den über Gleitrollen 60 geführten Seilzug 61 des Kolbens 19 der Kolbenpumpe 17 antreibt. Über ein in Fig. 3 dargestelltes Untersetzungsgetriebe 53, welches über ein Klinkenrad 54 eine Nockenwelle 55 antreibt, werden am Grundgehäuse 34 befestigte Quetschhebel 56 betätigt, wobei jeweils ein Quetschhebel 56 durch einen Nocken 57 der Nockenwelle 55 angehoben wird und eine der Zweigleitungen 7 zur Aufnahme einer Flüssigkeitsfraktion freigibt. Nach Aufnahme der Flüssigkeitsfraktionen wird jede Zweigleitung 7 nur an einer Stelle von einem Quetschhebel 56 abgeklemmt, wobei die in den Leitungen wirkenden Adhäsionskräfte ein Vermischen der Fraktionen bei Änderung der Lage der Kassette verhindern. Die einzelnen Nocken 57 zur Betätigung der Quetschhebel 56 sind im wesentlichen in zwei Schraubenlinien an der Nockenwelle 55 befestigt und weisen jeweils einen Abstand von 15° auf.

Zur Entnahme der einzelnen Flüssigkeitsfraktionen kann der Antrieb 21 der Kolbenpumpe 17 umgeschaltet werden, wobei - unter Beibehaltung der Drehrichtung der Nockenwelle - die zuerst gefüllte Zweigleitung auch zuerst wieder entleert wird.

Nach Abnahme der Kühleinrichtung 41 kann die Entleerung auch mit Hilfe eines mit der Saug- und Druckeinrichtung 3, 15 und der Steuereinrichtung 20 zusammenwirkenden Handrades oder Kurbel 58 vorgenommen werden.

Bei Probenentnahmen, welche nicht kontinuierlich erfolgen, ist es von Vorteil, wenn die Vorrichtung über eine Steuerelektronik verfügt, welcher über ein Display 59 ein individuelles Zeitsteuerprogramm eingegeben werden kann. Bei Stillstand der Saugeinrichtung 3 zwischen zwei Entnahmevorgängen, kann die Blutgerinnung an der im Blutstrom liegenden Nadel dadurch verhindert werden, daß mit einer kleinen Zusatzpumpe geringe Mengen Heparin zugeführt werden. Dazu könnte der Auffangbehälter 25 verwendet werden, welcher lediglich mit entsprechenden Antriebsmittel ausgestattet werden müßte.

## Patentansprüche

1. Vorrichtung zur Entnahme von Körperflüssigkeiten, mit einer Entnahmeeinrichtung (2), einer Saugeinrichtung (3), einer Druckeinrichtung (15) sowie einem Speichersystem (4) zur Aufnahme der Körperflüssigkeit, welches voneinander. trennbare Teilbereiche aufweist, welche die in bestimmten Zeitabschnitten gesammelten Flüssigkeitsfraktionen aufnehmen und eine eindeutige zeitliche Zuordnung der einzelnen Flüssigkeitsfraktionen zulassen, wobei die Entnahmeeinrichtung (2) als zweilumige Nadel ausgeführt ist, deren ein Lumen (13) mit der zuführenden (14) und deren anderes Lumen (11) mit der abführenden Leitung (6) verbunden ist, wobei die Saugeinrichtung (3) mit der abführenden Leitung (6) für die zu gewinnende Körperflüssigkeit und die zuführende Leitung (14) mit der Druckeinrichtung (15) in Verbindung steht, wobei weiters die Saugeinrichtung (3) und die Druckeinrichtung (15) durch eine Kolbenpumpe (17) realisiert sind, welche druckseitig ein mit der zuführenden Leitung (14) verbundenes Reservoir (16) für die in den Körper einzubringende Flüssigkeit und saugseitig einen mit dem Speichersystem (4) verbundenen Saugraum (10) aufweist.

2. Vorrichtung nach Anspruch 1, **dadurch gekennzeichnet,** daß ein Auffangbehälter (25) mit vorzugsweise variablem Fassungsvermögen vorgesehen ist, welcher über ein die Durchflußmenge steuerndes Organ (24) mit dem Reservoir (16) für die in den Körper einzubringende Flüssigkeit verbunden ist.

3. Vorrichtung nach Anspruch 2, **dadurch gekennzeichnet,** daß der Auffangbehälter (25) über Antriebsmittel verfügt, welche bei Stillstand der Saug- und Druckeinrichtung (3, 15) für eine kontinuierliche Zufuhr von Heparin zur Entnahmeeinrichtung (2) sorgen.

4. Vorrichtung zur Entnahme von Körperflüssigkeiten, mit einer Entnahmeeinrichtung (2), einer Saugeinrichtung (3), einer Druckeinrichtung (15) sowie einem Speichersystem (4) zur Aufnahme der Körperflüssigkeit, welches voneinander trennbare Teilbereiche aufweist, welche die in bestimmten Zeitabschnitten gesammelten Flüssigkeitsfraktionen aufnehmen und eine eindeutige zeitliche Zuordnung der einzelnen Flüssigkeitsfraktionen zulassen, wobei die Entnahmeeinrichtung (2) als zweilumige Nadel ausgeführt ist, deren ein Lumen (13) mit der zuführenden (14) und deren anderes Lumen (11) mit der abführenden Leitung (6) verbunden ist, wobei die Saugeinrichtung (3) mit der abführenden Leitung (6) für die zu gewinnende Körperflüssigkeit und die zuführende Leitung (14) mit der Druckeinrichtung (15) in Verbindung steht, wobei weiters der Antrieb (21) der Saug- und Druckeinrichtung (3, 15), sowie jener der Steuereinrichtung (20) des Speichersystems (4) gemeinsam mit der zugehörigen Steuerelektronik (35) in einem Grundgehäuse (34) angeordnet ist und das Speichersystem (4) zur Aufnahme der Körperflüssigkeit, die Saug- und Druckeinrichtung (3, 15) sowie deren Verbindungsleitungen (5, 8) in einer vom Grundgehäuse (34) abnehmbaren Kassette (36) angeordnet sind.

5. Vorrichtung nach Anspruch 4, **dadurch gekennzeichnet,** daß die Kassette (36) U-förmig ausgebildet ist, wobei an beiden Seitenflächen (37, 38) des Grundgehäuses (34) je ein Teil (39, 40) der Kassette mit jeweils einer Vielzahl einzelner Zweigleitungen (7) vorgesehen ist.

6. Vorrichtung nach Anspruch 5, **dadurch gekennzeichnet,** daß alle Zweigleitungen (7) über eine Sammelleitung (8) mit der Saugeinrichtung (3) verbunden sind.

7. Vorrichtung nach Anspruch 4, **dadurch gekennzeichnet,** daß die das Speichersystem (4) aufnehmende Kassette (36) bzw. deren Teile (39, 40) mit einer regelbaren Kühleinrichtung (41) in Verbindung stehen, welche über ein Gehäuse (42) zur Aufnahme eines Kühlgels (43) verfügt und in der dem Speichersystem (4) zugewandten Wand (45) vorzugsweise gleichmäßig verteilte Öffnungen (46) aufweist , über derer durch einen Thermostat (49) steuerbaren Öffnungsquerschnitte Kühlluft zum Speichersystem (4) zutritt.

8. Vorrichtung nach Anspruch 5, **dadurch gekennzeichnet,** daß ein Antriebsmotor (50) vorgesehen ist, welcher über ein Spindelgetriebe (51) die Saug- und Druckeinrichtung (3, 15) betätigt und über ein Untersetzungsgetriebe (53) und ein Klinkenrad (54) eine Nockenwelle (35) antreibt, deren Nocken (57) mit am Grundgehäuse (34) befestigten Quetschhebeln (56) der Steuereinrichtung (20) zusammenwirken, wobei jeweils ein durch einen Nocken (57) angehobener Quetschhebel (56) eine der Zweigleitungen (7) freigibt.

9. Vorrichtung nach Anspruch 1 oder 4, **dadurch gekennzeichnet,** daß der Antrieb (21) der Saug- und Druckeinrichtung (3, 15) zur Entnahme der einzelnen Flüssigkeitsfraktionen aus dem Speichersystem (4) umschaltbar ist.

10. Vorrichtung nach Anspruch 1 oder 4, **dadurch gekennzeichnet,** daß zur Entnahme der einzelnen Flüssigkeitsfraktionen ein mit der Saug- und Druckeinrichtung (3, 15) und der Steuereinrichtung (20) des Speichersystems (4) zusammenwirkendes Handrad bzw. Kurbel (58) vorgesehen ist.

11. Vorrichtung nach Anspruch 1 oder 4, **dadurch gekennzeichnet,** daß der Durchmesser der die Flüssigkeitsfraktionen aufnehmenden Zweigleitungen (7) bzw. des Schlauches (26) klein bemessen ist, sodaß zwischen den Innenwänden des Speichersystems (4) und der Körperflüssigkeit wirkende Adhäsionskräfte größer' sind, als von außen auf die Vorrichtung wirkende Kräfte, wie Schwerkraft oder zusätzliche Beschleuigungskräfte.

## Claims

1. A device for taking samples of body fluids, comprising a sampling unit (2), a suction unit (3), a pressure unit (15) and a storage system (4) receiving the body fluid, which system (4) contains individual areas that may be separated from each other and will hold the fluid fractions gathered at certain intervals, permitting the individual fluid fractions to be clearly identified with regard to their time of withdrawal, where the sampling unit (2) is configured as a double-lumen needle, one lumen (13) being connected to the ingoing line (14) and the other lumen (11) to the outgoing line (6), and the suction unit (3) being connected to the outgoing line (6) for the body fluid to be gathered, and the ingoing line (14) being connected to the pressure unit (15), and where the suction unit (3) and the pressure unit (15) are combined in a plunger pump (17), which is provided on the pressure side with a reservoir (16) connected to the ingoing line (14) and containing the fluid to be introduced into the body, and on the suction side with a suction chamber (10) connected to the storage system (14).

2. A device as in claim 1, **characterized in that** a collecting vessel (25) is provided, preferably of variable capacity, which is connected via a flow-regulating element (24) to the reservoir (16) for the fluid to be introduced into the body.

3. A device as in claim 2, **characterized in that** the collecting vessel (25) is provided with a driving means via which the sampling unit (2) is continuously supplied with heparin during a standstill of the suction and pressure unit (3, 15).

4. A device for taking samples of body fluids, comprising a sampling unit (2), a suction unit (3), a pressure unit (15) and a storage system (4) receiving the body fluid, which system (4) contains individual areas that may be separated from each other and will hold the fluid fractions gathered at certain intervals, permitting the individual fluid fractions to be clearly identified with regard to their time of withdrawal, where the sampling unit (2) is configured as a double-lumen needle, one lumen (13) being connected to the ingoing line (14) and the other lumen (11) to the outgoing line (6), and the suction unit (3) being connected to the outgoing line (6) for the body fluid to be gathered, and the ingoing line (14) being connected to the pressure unit (15), and where the drive (21) of the suction and pressure unit (3, 15) as well as that of the control device (20) of the storage system (4), together with the corresponding control electronics (35) is located in a main housing (34), and where the storage system (4) receiving the body fluid, and the suction and pressure unit (3, 15) along with the connecting lines (5, 8) are located in a case (36) which is detachable from the main housing (34).

5. A device as in claim 4, **characterized in that** the case (36) is U-shaped, its halves (39, 40) being situated one on either side (37, 38) of the main housing (34), each including a number of individual branch lines (7).

6. A device as in claim 5, **characterized in that** all branch lines (7) are connected to the suction unit (3) by means of a manifold (8).

7. A device as in claim 4, **characterized in that** the case (36) containing the storage system (4), or rather, its halves (39, 40) are connected to an adjustable cooling unit (41), which has a container (42) for a cooling gel (43), and whose wall (45) facing the storage system (4) is provided with preferably regularly spaced openings (46) whose cross-sections, which are controlled by a thermostat (49), will admit cool air into the storage system.

8. A device as in claim 5, **characterized in that** a driving motor (50) is provided which actuates the suction and pressure unit (3, 15) by means of a spindle drive (51), and drives a camshaft (35) via a reducing gear (53) and a ratchet wheel (54), the cams (57) of the said camshaft (35) cooperating with squeezing levers (56) of the control device (20) attached to the main housing (34), each such cam-lifted lever (56) giving access to one of the branch lines (7).

9. A device as in claim 1 or 4, **characterized in that** the drive (21) of the suction and pressure unit (3, 15) is reversible in order to permit removal of the individual fluid fractions from the storage system (4).

10. A device as in claim 1 or 4, **characterized in that** the individual fluid fractions are withdrawn using a knob or crank (58) cooperating with the suction and pressure unit (3, 15) and the control unit (20) of the storage system (4).

11. A device as in claim 1 or 4, **characterized in that** the diameters of the branch lines (7) holding the fluid fractions, or rather, of the flexible tube (26) are kept small, such that adhesive forces acting between the inner walls of the storage system (4) and the body fluid are larger than exterior forces acting upon the device, such as gravitation or additional accelerating forces.

## Revendications

1. Dispositif de prélèvement de liquides corporels, comprenant un dispositif de prélèvement (2) un dispositif d'aspiration (3), un dispositif de compression (15) ainsi qu'un dispositif de stockage (4) pour la réception du liquide corporel, qui comporte des zones partielles séparables les unes des autres qui permettent de prélever les fractions de liquide recueillies dans des laps de temps déterminés et dans un ordre dans le temps sans équivoque, le dispositif de prélèvement (2) est réalisé sous forme d'aiguille à deux lumières, dont une lumière (13) est reliée au canal d'arrivée (14) et dont l'autre lumière (11) est reliée au canal de sortie (6), le dispositif d'aspiration (3) étant relié au canal de sortie (6) pour recueillir le liquide corporel et le canal d'entrée (14) avec le dispositif de compression (15), en outre le dispositif d'aspiration (3) et le dispositif de compression (15) sont matérialisés par une pompe à piston (17) qui présente côté charge un réservoir (16) branché au canal d'arrivée (14) pour le liquide à injecter dans le corps et côté aspiration une chambre d'aspiration (10) branchée au dispositif de stockage (4).

2. Dispositif selon la revendication 1, caractérisé en ce qu'un récipient de collecte (25) est muni de possibilités de captage variables, ce récipient étant relié par un organe (24) commandant la quantité de liquide en écoulement avec le réservoir (16) pour le liquide à introduire dans le corps.

3. Dispositif selon la revendication 2, caractérisé en ce que le récipient de collecte (25) dispose d'un moyen d'entraînement qui, en période de repos du système d'aspiration-compression (3, 15), pour-voit à une amenée continue d'héparine dans le dispositif de prélèvement (2).

4. Dispositif de prélèvement de liquides corporels, comprenant un dispositif de prélèvement (2), un dispositif d'aspiration (3), un dispositif de compression (15) ainsi qu'un dispositif de stockage (4) pour la réception du liquide corporel, qui comporte des zones partielles séparables les unes des autres qui permettent de prélever les fractions de liquide recueillies dans des laps de temps déterminés et dans un ordre dans le temps sans équivoque, le dispositif de prélèvement (2) est réalisé sous forme d'aiguille à deux lumières, dont une lumière (13) est reliée au canal d'arrivée (14) et dont l'autre lumière (11) est reliée au canal de sortie (6), le dispositif d'aspiration (3) étant relié au canal de sortie (6) pour recueillir le liquide corporel et le canal d'entrée (14) au dispositif de compression (15), caractérisé en ce que l'entraînement (21) du dispositif d'aspiration-compression (3, 15), ainsi que chaque dispositif de commande (20) du dispositif de stockage (4) est associé en commun avec l'électronique de commande propre (35) dans un carter de base (34), et en ce que le dispositif de stockage (4) pour la réception du liquide corporel, le dispositif d'aspiration-compression (3, 15) ainsi que les canaux de liaisons (5, 8) sont placés dans une cassette (36) séparable du carter de base (34).

5. Dispositif selon la revendication 4, caractérisé en ce que la cassette (36) présente une forme en U, où, sur les deux surfaces latérales (37, 38) du carter (34), est prévue une partie de la cassette (39, 40) comportant chacune de nombreux canaux de dérivation séparés (7).

6. Dispositif selon a revendication 5, caractérisé en ce que tous les canaux de dérivation (7) sont reliés par un canal collecteur (8) au dispositif d'aspiration.

7. Dispositif selon la revendication 4, caractérisé en ce que la cassette (36), ou ses portions (39, 40) recevant le dispositif de stockage (4), est en communication avec un dispositif réfrigérant (41) réglable qui dispose d'un logement pour la réception d'un gel réfrigérant (43) et comporte avantageusement dans la paroi (45) faisant face au dispositif de stockage (4), des ouvertures (46) réparties régulièrement, et par leur section de passage réglable par un thermostat (49) passe l'air de refroidissement vers le dispositif de stockage (4).

8. Dispositif selon la revendication 5, caractérisé en ce qu'il est prévu un moteur d'entraînement (50) qui actionne, par un engrenage à vis (51), le dispositif d'aspiration et de compression (3, 15) et entraîne, par l'intermédiaire d'un démultiplicateur (53) et d'une roue à rochet (54) un arbre à cames (35) dont les cames (57) coopèrent avec des leviers d'échappement (56) du dispositif de commande (20) fixés sur le carter de base (34), où chaque levier d'échappement (56) soulevé par une came (57) libère un des canaux de dérivation (7).

9. Dispositif selon la revendication 1 ou 4, caractérisé en ce que l'entraînement (21) du système d'aspiration-compression (3, 15) pour le prélèvement de fractions liquides séparées du dispositif de stockage est de sens inversable.

10. Dispositif selon la revendication 1 ou 4, caractérisé en ce que pour le prélèvement de fractions liquides séparées, on prévoit un volant ou une manivelle (58) coopérant avec le dispositif d'aspiration-compression (3, 15) et le dispositif de stockage (4).

11. Dispositif selon la revendication 1 ou 4, caractérisé en ce que le diamètre des canaux de dérivation (7) ou des tuyaux souples (26) recevant les fractions de liquide, est d'une dimension faible, de sorte qu'entre les parois intérieures du dispositif de stockage (4) et le liquide corporel, les forces d'adhésion sont plus grandes, que les forces agissant de l'extérieur sur le dispositif, telles que la gravité ou les forces d'accélération additionnelles.
